# EUROPEAN PATENT APPLICATION

(11) **EP 2 522 275 A1**
(43) Date of publication of application: **14.11.2012**
(21) Application number: 12167356.0
(22) Date of filing: 09.05.2012
(51) Int. Cl.: A61B 5/08, A61B 5/083, A61B 5/097, A61M 16/08

(54) **Water trapping apparatus**

(30) Priority: 09.05.2011 JP 2011104502
(71) Applicant: Nihon Kohden Corporation, Shinjuku-ku Tokyo (JP)
(72) Inventor: Kobayashi, Naofumi, Tokyo (JP); Yamamori, Shinji, Tokyo (JP); Suzuki, Katsuyoshi, Tokyo (JP)
(74) Representative: Ilgart, Jean-Christophe

(57) **Abstract**

A water trapping apparatus includes: a respiratory gas contacting portion that is disposed in a flow path through which a respiratory gas flows, and that is adapted to be in contact with the respiratory gas, the respiratory gas contacting portion that is comprised of a porous body; and an ambient-air contacting portion that is formed integrally with the respiratory gas contacting portion, and that is adapted to be in contact with ambient air, the ambient-air contacting portion that is comprised of the porous body.

## Description

### BACKGROUND OF THE INVENTION

The present invention relates to a water trapping apparatus which is to be disposed in a part of a flow path through which a respiratory gas flows, and which can be used in a continuous measurement of the respiratory gas.

In a measurement of a respiratory gas by the side stream method (the sampling method), part of the respiratory gas is taken in through a sampling tube, and therefore water contained in the respiratory gas is liquefied in the sampling tube and a measuring apparatus, thereby raising the possibility of disturbing the measurement. In some cases, there is the possibility that the measuring apparatus breaks down.

Therefore, a water trap functioning as a water trapping apparatus is placed in front of a measuring apparatus, and water is separated. However, there is a case where a path through which a respiratory gas is guided to the measuring apparatus is complicated by the water trap, and moreover problems arise such as that a pressure must be applied so as to adequately accumulate liquefied water and the like into the water trap. Furthermore, for example, a cooling mechanism for properly advancing liquefaction in the water trap is provided, thereby causing problems in that the structure is complicated, and that the production cost is increased.

In contrast with such a complicated water trap, there is a configuration where the outer circumference of a gas sampling tube is covered by a hydrophilic member, and further by a housing of a drying material, bacteria and the like are captured by a hydrophobic member disposed in a middle of a gas sampling line, and water is guided toward the housing by the capillary phenomenon of the hydrophilic member, thereby preventing water from entering a measuring apparatus (see W02010/030226A).

### SUMMARY

It is therefore an object of the invention to provide a water trapping apparatus in which water contained in a respiratory gas can be trapped by a simple and economical configuration for a relatively long term without requiring replacement.

In order to achieve the object, according to the invention, there is provided a water trapping apparatus comprising: a respiratory gas contacting portion that is disposed in a flow path through which a respiratory gas flows, and that is adapted to be in contact with the respiratory gas, the respiratory gas contacting portion that is comprised of a porous body; and an ambient-air contacting portion that is formed integrally with the respiratory gas contacting portion, and that is adapted to be in contact with ambient air, the ambient-air contacting portion that is comprised of the porous body.

The water trapping apparatus may further comprise a gas reservoir to which an inflow tube into which the respiratory gas flows and an outflow tube from which the respiratory gas, which has flowed in the gas reservoir, flows are connected, the gas reservoir holding the respiratory gas contacting portion in a state where the respiratory gas contacting portion faces to the gas reservoir.

The inflow tube and the outflow tube may be connected to a ceiling side of the gas reservoir, and the respiratory gas contacting portion may be disposed on a bottom side which is opposed to the ceiling side.

The bottom side of the gas reservoir may be closer to the inflow tube than the outflow tube.

A partition plate may be disposed between the inflow tube and the outflow tube.

A metal member may be inserted into at least one of the inflow tube and the outflow tube.

The porous body may have a hollow shape configured by an inner wall portion, which is the respiratory gas contacting portion, and an outer circumferential portion, which is the ambient-air contacting portion.

The water trapping apparatus may be disposed in an exhaust port of an artificial ventilator.

### BRIEF DESCRIPTION OF THE DRAWINGS

Fig. 1 is a block diagram showing an example of a medical system which uses a water trapping apparatus of an embodiment of the invention.
Fig. 2 is a perspective view showing a water trapping apparatus of a first embodiment of the invention.
Fig. 3 is a sectional view taken along line A-A in Fig. 2.
Fig. 4 is a perspective view showing a water trapping apparatus of a second embodiment of the invention.
Fig. 5 is a front view showing the water trapping apparatus of the second embodiment of the invention.
Fig. 6 is a sectional view taken along line B-B in Fig. 5.

### DETAILED DESCRIPTION OF EMBODIMENTS

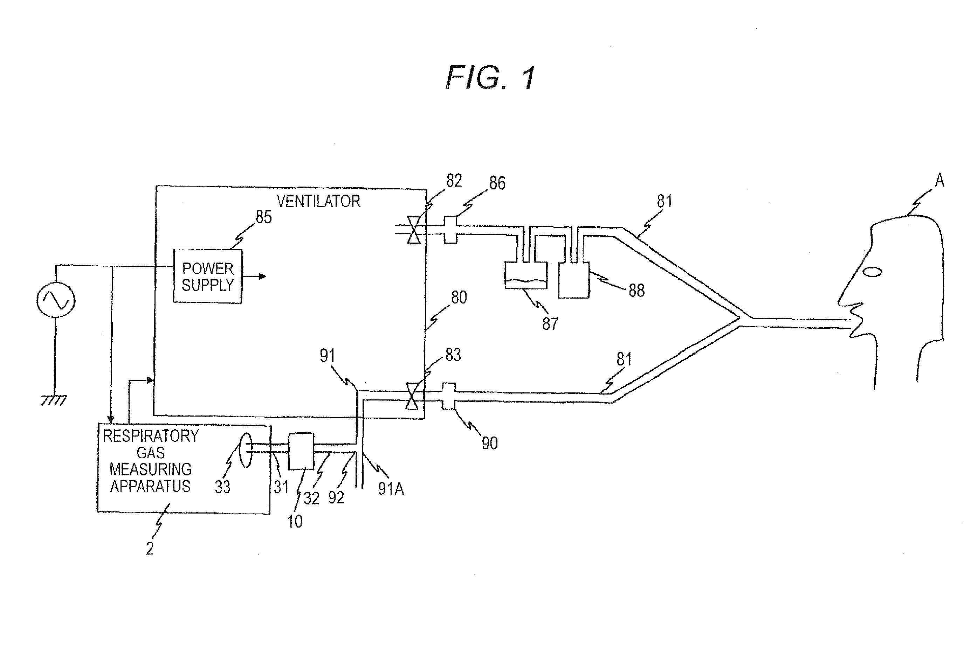

Hereinafter, a water trapping apparatus of an embodiment of the invention will be described with reference to the accompanying drawings. In the figures, the identical components are denoted by the same reference numerals, and duplicated description will be omitted. The water trapping apparatus 10 of the embodiment can be used in, for example, a medical system which is shown in Fig. 1, and which uses an artificial ventilator 80 and a respiratory gas measuring apparatus 2.

The ventilator 80 is connected to the respiratory system of the patient A through an inspiratory valve 82 which is a one-way valve, an infection prevention filter 86, a heating and humidifying device 87, a water trap 88, and a respiratory circuit 81. The respiratory system of the patient A is connected to an exhaust pipe 91 of the ventilator 80 through the respiratory circuit 81, an infection prevention filter 90, and an expiratory valve 83 which is a one-way valve. A respiratory gas is sent from the ventilator 80 to the patient through the inspiratory valve 82, and the respiratory gas of the patient is discharged from an exhaust port 91A through the expiratory valve 83 and the exhaust pipe 91.

In the medical system including the ventilator 80, the infection prevention filters 86, 90, the heating and humidifying device 87, the water trap 88, and the respiratory circuit 81 are configured so as to be easily attached and detached, in order to enable replacement, sterilization, or replacement for every patient.

The ventilator 80 includes a power supply 85 which supplies an electric power to various parts of the ventilator 80. Some of simple ventilators which are operated manually or by an additional pressure source or the like do not include a power supply.

A branch fitting 92 is formed in the exhaust port 91A. A sampling tube 31 which is an inflow tube is connected to the branch fitting 92, and also to the water trapping apparatus 10. A sampling tube 32 which is an outflow tube is connected to the water trapping apparatus 10, and also to the respiratory gas measuring apparatus 2 through a bacteria filter 33.

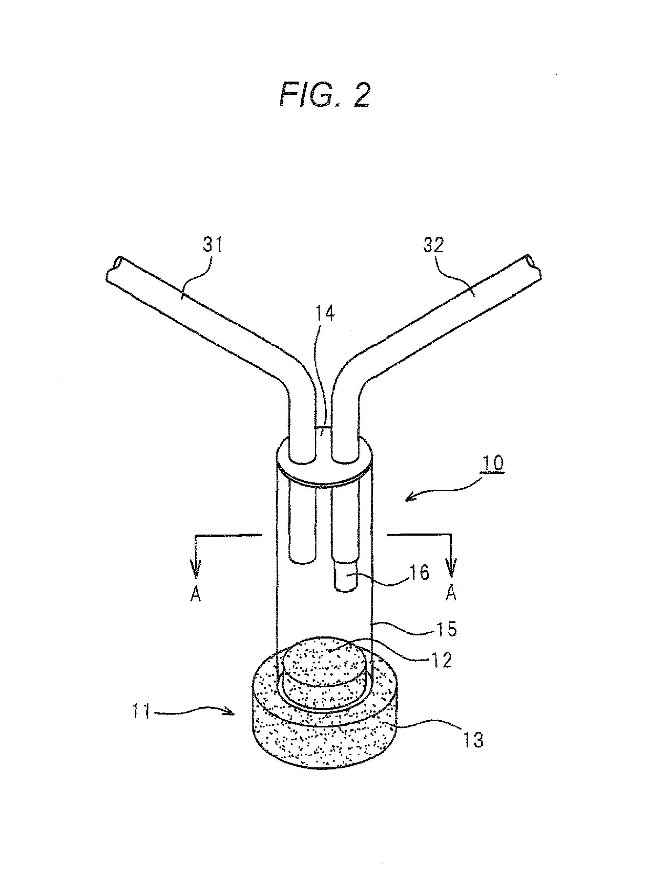

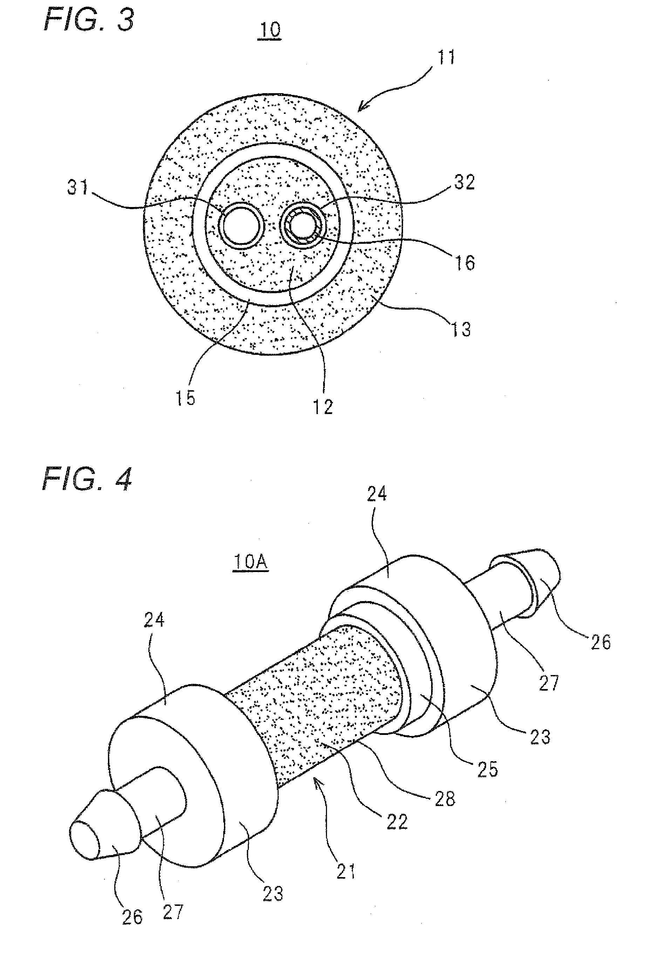

The water trapping apparatus 10 of the first embodiment is configured as shown in Figs. 2 and 3. Namely, the water trapping apparatus 10 of the first embodiment includes a porous body 11 having a shape in which two disk plates are integrally stacked on each other. The diameter of the upper disk plate 12 of the porous body 11 is shorter than that of the lower disk plate 13.

For example, the porous body 11 is formed by molding polyethylene in which the surface is hydrophilicated, into a porous body by sinter forming. A porous body which is manufactured by ASAHI KASEI CHEMICALS CORPORATION under the registered trademark Sunfine may be used as the porous body 11.

The water trapping apparatus 10 includes a gas reservoir 15 which has a top plate 14 in the head part (ceiling), and in which the bottom part is opened. The gas reservoir 15 has a cylindrical shape, but may have any shape. The upper disk plate 12 of the porous body 11 is fitted to the opening of the gas reservoir 15, and the lower disk plate 13 is used as a stopper, so that the whole porous body 11 functions as a lid of the gas reservoir 15.

As a result, the disk plate 12 functions as a respiratory gas contacting portion which is to be in contact with a respiratory gas. Furthermore, the disk plate 13 functions as an ambient-air contacting portion which is formed integrally with the respiratory gas contacting portion, and which is to be in contact with the ambient air. The gas reservoir 15 holds the disk plate 12 which is the respiratory gas contacting portion, in a state where the disk plate 12 which is the respiratory gas contacting portion faces toward the interior of the gas reservoir 15.

In the sampling tube 32, as shown in Fig. 2, a hollow metal member 16 having a cylindrical shape is inserted into a tip end part which is inserted into the gas reservoir 15, to be held thereby. Although not shown, alternatively, a rod-like metal member 16 may be inserted into the tip end part to be held by friction with the inner wall. A gap exists between the metal member 16 and the inner wall of the sampling tube 32, and therefore the sampling tube 32 is not blocked by the metal member 16. Although, in Fig. 2, the metal member 16 is inserted into the sampling tube 32, the metal member may be inserted into the sampling tube 31, or into both the sampling tubes 31, 32.

In the operating state of the system, the thus configured water trapping apparatus 10 is preferably used in a state where, as shown in Fig. 1, the apparatus is hung from the sampling tubes 31, 32 and the porous body 11 is positioned in the lowest part. In this state, during a process from inflowing into the gas reservoir 15, to outflowing from the sampling tube 31, the respiratory gas arriving from the sampling tube 32 is cooled particularly inside the gas reservoir 15 and in the vicinity of the place of the sampling tube 31 where the metal member 16 is disposed, and water contained in the respiratory gas is liquefied.

Although not illustrated, the lengths of the sampling tubes 31, 32 in the gas reservoir 15 may be different from each other. Specifically, it is preferred that the distance between the sampling tube 32 and the disk plate 12 is shorter than that between the sampling tube 31 and the disk plate 12. Although a negative pressure is applied to the sampling tube 31, the difference in length prevents liquefied liquid in the sampling tube from being sucked into the sampling tube 31. Although not illustrated, it is further preferred that a partition plate may be disposed between the sampling tubes 31, 32 so as to prevent liquefied liquid from being sucked into the sampling tube 31.

The liquefied liquid is adsorbed into the disk plate 12 of the porous body 11, and penetrates into the lower disk plate 13. Since most of the surface of the disk plate 13 is in contact with the ambient air, the liquefied liquid which has penetrated into the disk plate is evaporated. Therefore, the porous body 11 continues to perform evaporation of water while trapping water, and hence the porous body 11 can be used for a relatively long term.

In the respiratory gas which is sent to the respiratory gas measuring apparatus 2 through the sampling tube 31 and the bacteria filter 33, water has been trapped in the water trapping apparatus 10. Therefore, dew condensation does not occur in the respiratory gas measuring apparatus 2. For example, the respiratory gas measuring apparatus 2 includes a carbon dioxide concentration sensor or the like. The sensor receives the arriving respiratory gas, measures the concentration of carbon dioxide, and outputs a result from outputting means (not shown) such as a displaying device. The output of the carbon dioxide concentration sensor of the respiratory gas measuring apparatus 2 is sent to the ventilator 80, so that the EtCO2 of the patient A is measured, and the situation of the gas exchange of the patient A is monitored. When the suction pressure in the sampling tube is increased, there is the possibility that the respiratory gas in the water trapping apparatus 10 leaks from the porous body 11. However, it is possible to select a suction pressure at which most of the respiratory gas can be guided from the water trapping apparatus 10 to the respiratory gas measuring apparatus 2 through the sampling tube 31, and a measurement (particularly, a measurement of the respiratory gas in the exhaust port of the ventilator) can be performed without causing any particular problem in the respiratory gas measuring apparatus 2 in which severe accuracy is not requested.

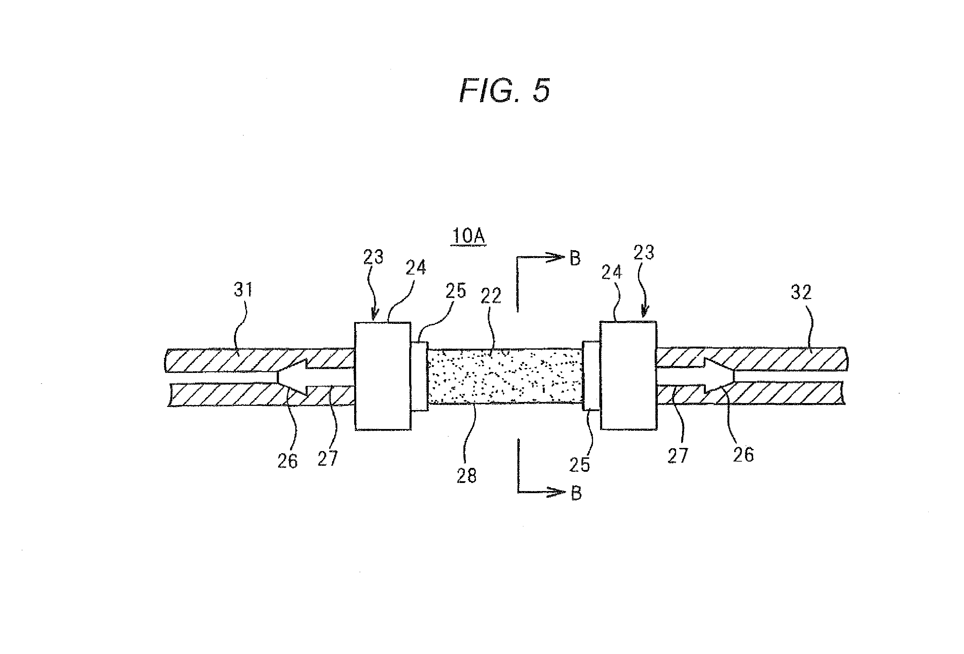

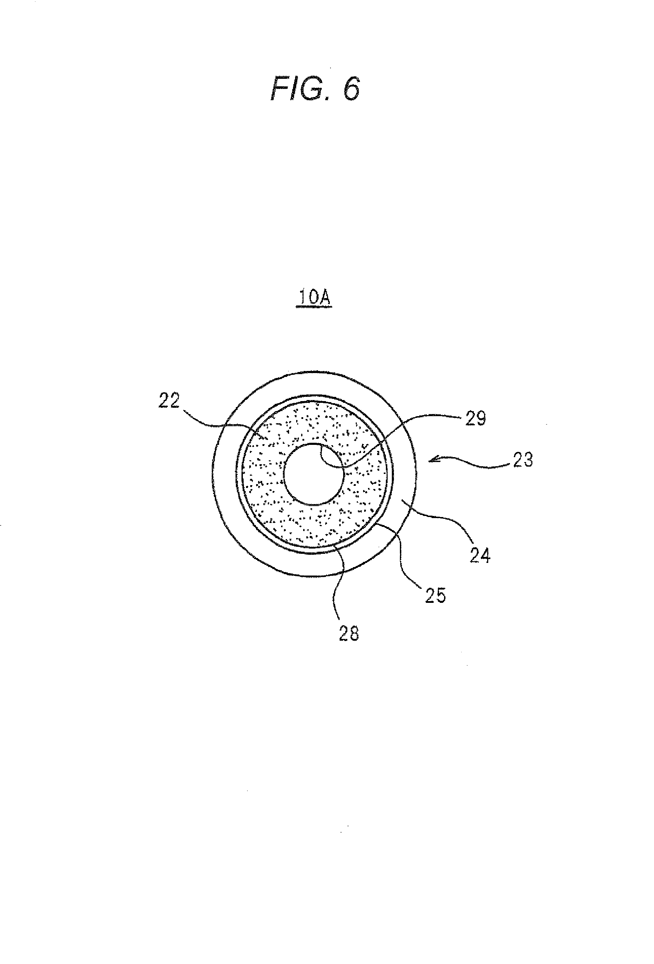

Figs. 4 to 6 show a water trapping apparatus 10A of a second embodiment. In the water trapping apparatus 10A, a porous body 21 in the middle is configured by a hollow cylinder body 22. In the embodiment, the cylinder body 22 has a circular cylindrical shape. Alternatively, the cylinder body may be rectangularly cylindrical, straight in the longitudinal direction, or bent.

In the ends of the cylinder body 22, sleeves 23 are fitted to end portions of the cylinder body 22. The sleeves 23 are made of, for example, a metal. In each of the sleeves, a middle portion 24 has a larger diameter, and the portion into which the end portion of the cylinder body 22 is inserted is formed as a frame portion 25 which is smaller in diameter than the middle portion 24. In the side of the middle portion 24 opposite to the frame portion 25, a hollow fitting portion 27 in which a retaining portion 26 having a tapered shape is formed in the tip end is formed. The fitting portion 27 can be easily inserted into end portions of the sampling tube 31 or 32.

An outer circumferential portion 28 of the cylinder body 22 functions as an ambient-air contacting portion, and an inner wall portion 29 facing the hole portion of the cylinder body 22 functions as a respiratory gas contacting portion. The water trapping apparatus 10A of the second embodiment can be used while being placed in the position of the water trapping apparatus 10 shown in Fig. 1. In this case, in the course extending from the entering of the respiratory gas arriving from the sampling tube 32 into the sleeve 23 on the entrance side, to the flow out from the sleeve 23 on the exit side through the cylinder body 22, the sleeve 23 may be made of a metal. In the configuration, the respiratory gas is cooled in the vicinity of the place where the sleeve 23 is disposed, and water contained the respiratory gas is liquefied.

The liquefied liquid is adsorbed into the inner wall portion 29 of the cylinder body 22 of the porous body 21, and penetrates into the outer circumferential portion 28 on the outer side. In this case, when the cylinder body 22 is bent into a V-like shape extending from the middle part to the both sides, and used in this posture, it is expected that penetration of water from the sleeve 23 to the cylinder body 22 satisfactorily advances. Since the surface of the outer circumferential portion 28 is in contact with the ambient air, the penetrating liquid is evaporated. Therefore, the porous body 21 continues to perform evaporation of water while trapping water, and hence the porous body 21 can be used for a relatively long term.

According to an aspect of the invention, the contacting portions are configured by a porous body. When water contained in a respiratory gas is liquefied, therefore, the water can be appropriately trapped and held. Since the ambient-air contacting portion is configured integrally with the respiratory gas contacting portion and by a porous body, moreover, the trapped water moves to the ambient-air contacting portion to be evaporated. Therefore, evaporation is enabled while trapping of water is performed, and hence the apparatus can be used for a relatively long term. Since the water trapping apparatus has a simple structure, the apparatus configuration is simplified, and attachment is easily performed. Therefore, the apparatus can be handled very easily.

According to an aspect of the invention, in the case where the respiratory gas flows into the gas reservoir through the inflow tube, and the respiratory gas is guided from the gas reservoir to a measuring apparatus through the outflow tube, the gas reservoir holds the respiratory gas contacting portion in the state where the respiratory gas contacting portion faces to the gas reservoir. Therefore, water is trapped in the gas reservoir, and absorbed into the respiratory gas contacting portion.

According to an aspect of the invention, water of the respiratory gas which flows into the gas reservoir from the ceiling side is trapped, and caused to be absorbed into the respiratory gas contacting portion on the bottom side by gravity, so that trapping of water is adequately performed.

According to an aspect of the invention, the inflow tube is disposed on the bottom side with respect to the outflow tube. In the case where liquefied water is discharged from the inflow tube, even when a negative pressure is applied to the outflow tube, therefore, the water is not erroneously sucked into the outflow tube.

According to an aspect of the invention, the partition plate is disposed between the inflow tube and the outflow tube. In the case where liquefied water is discharged from the inflow tube, therefore, the water is not erroneously sucked into the outflow tube.

According to an aspect of the invention, the metal member is inserted into at least one of the inflow tube and the outflow tube. Therefore, the respiratory gas in the vicinity of the metal member is rapidly cooled, and efficient trapping of water is enabled.

According to an aspect of the invention, water is trapped and absorbed by the respiratory gas contacting portion in the inner wall portion. The trapped water moves to the ambient-air contacting portion to be evaporated. Therefore, evaporation is enabled while trapping of water is performed, and hence the apparatus can be used for a relatively long term.

## Claims

1. A water trapping apparatus comprising:
a respiratory gas contacting portion that is disposed in a flow path through which a respiratory gas flows, and that is adapted to be in contact with the respiratory gas, the respiratory gas contacting portion that is comprised of a porous body; and
an ambient-air contacting portion that is formed integrally with the respiratory gas contacting portion, and that is adapted to be in contact with ambient air, the ambient-air contacting portion that is comprised of the porous body.

2. The water trapping apparatus according to claim 1, further comprising a gas reservoir to which an inflow tube into which the respiratory gas flows and an outflow tube from which the respiratory gas, which has flowed in the gas reservoir, flows are connected, the gas reservoir holding the respiratory gas contacting portion in a state where the respiratory gas contacting portion faces to the gas reservoir.

3. The water trapping apparatus according to claim 2,
wherein
the inflow tube and the outflow tube are connected to a ceiling side of the gas reservoir, and
the respiratory gas contacting portion is disposed on a bottom side which is opposed to the ceiling side.

4. The water trapping apparatus according to claim 3, wherein the bottom side of the gas reservoir is closer to the inflow tube than the outflow tube.

5. The water trapping apparatus according to claim 2, wherein a partition plate is disposed between the inflow tube and the outflow tube.

6. The water trapping apparatus according to claim 2, wherein a metal member is inserted into at least one of the inflow tube and the outflow tube.

7. The water trapping apparatus according to claim 1, wherein the porous body has a hollow shape configured by an inner wall portion, which is the respiratory gas contacting portion, and an outer circumferential portion, which is the ambient-air contacting portion.

8. The water trapping apparatus according to claim 1, which is disposed in an exhaust port of an artificial ventilator.
